# EUROPEAN PATENT SPECIFICATION

(11) **EP 3 313 328 B1**
(45) Date of publication and mention of the grant of the patent: **27.05.2020**
(21) Application number: 16756793.2
(22) Date of filing: 24.06.2016
(51) Int. Cl.: A61F 2/74, B25J 9/14, F15B 15/10, A61F 2/68, A61F 2/50

(54) **PNEUMATIC DEVICE FOR ACTUATING ORGANS**
PNEUMATISCHE VORRICHTUNG ZUR BETÄTIGUNG VON ORGANEN
DISPOSITIF PNEUMATIQUE POUR ACTIVER DES ORGANES

(30) Priority: 26.06.2015 IT UB20151717 U
(43) Date of publication of application: 02.05.2018
(73) Proprietor: Scuola Superiore Sant'Anna, 56127 Pisa (IT); Fondazione Istituto Italiano di Tecnologia, 16163 Genova (IT)
(72) Inventor: CIANCHETTI, Matteo, 56127 Pisa (IT); LASCHI, Cecilia, 56127 Pisa (IT); DARIO, Paolo, 56127 Pisa (IT); SHAH, Syed Taimoor Hassan, 56127 Pisa (PI) (IT); MAZZOLAI, Barbara, 16163 Genova (IT)
(74) Representative: Leotta, Antonio
(86) International application number: PCT/IB2016/053786
(87) International publication number: WO 2016/207855

(56) References cited:
- WO-A2-2015/051380
- US-A- 5 067 390
- US-A- 5 158 005
- US-A- 5 165 323
- KRISHNAN GIRISH: "Kinematics of a new class of smart actuators for soft robots based on generalized pneumatic artificial muscles", 2014 IEEE/RSJ INTERNATIONAL CONFERENCE ON INTELLIGENT ROBOTS AND SYSTEMS, IEEE, 14 September 2014 (2014-09-14), pages 587-592, XP032676710, DOI: 10.1109/IROS.2014.6942619

## Description

### Technical Field

The present invention relates to the technical sector concerning actuating devices able to impart movements to bodies.

In the cited technical field are present various types of such actuating devices, commonly used in mechanical applications and in robotics.

In particular, the invention relates to the devices known as artificial muscles or pneumatic muscles.

A pneumatic actuation device in accordance with the preamble of claim 1 is known from prior art document US 5,067,390 A.

### Background Art

In these devices, the mechanical work is not obtained from the movement of a piston in a cylinder but, conceptually, by exploiting the elastic deformation of rooms that are pressurized with air or other fluid.

The substantial difference lies in the fact that while a conventional pneumatic actuator has a 'rigid' behavior, that means it implements the movement only in the predetermined mode, for example rectilinear, in an artificial muscle the behavior can vary according to the type of application and It may allow deviations of the thrust directions, or of shooting, because of external events, foreseen or unforeseen.

In the prior art document US2003 / 0205045A1, the artificial muscle comprises an elastic central cylindrical chamber, interposed between two head members, with the latter ones guided, for example, in the axial direction.

The side surface of said elastic central cylindrical chamber is circumscribed by an annular elastic chamber, whose internal wall is constituted by the same central chamber.

The two chambers are interconnected by a closed circuit pneumatic plant, comprising a connecting duct and a bi-directional pump.

By actuating the pump so that the fluid is removed from the cylindrical central chamber and placed in the annular chamber, a mutual approaching of the head members is obtained, that means a contraction of the artificial muscle.

By reversing the drive direction of the pump, of course, an opposite effect is obtained, with the mutual spacing of the head members, that means an extension of the artificial muscle.

The technique solution described is a relatively simple solution in terms of the operating principle, but in practice the need to have a closed circuit pneumatic system, with relative pump, determines both not negligible undesired dimensions and constructional complexity.

Similar devices are also known as McKibben muscles, in which is provided a cylindrical hollow chamber, tight, made of elastomeric material, adapted to be pressurized with air or other fluid by by means of blowing, for example external.

On the outside of said hollow cylindrical chamber is coaxially disposed a braided sleeve, consisting of a first set of flexible and substantially inextensible wires, arranged each according to its own helical path of the same diameter and helix angle but different principles and by a second set of the same flexible and substantially inextensible wires, arranged according to respective helical paths symmetric to those of the above mentioned first set.

The said first and second sets of flexible wires, therefore, form the mesh of the above said sleeve, in which each of said helical paths is inclined to a same predetermined angle with respect to the longitudinal axis of said device.

The device also comprises two rigid head members, provided at the corresponding ends of the braided sleeve, to which are attached both the head members of the respective threads and the cylindrical hollow chamber.

The rigid head members are adapted to be mechanically connected to external users means.

In an active phase of the device, the blow-in bodies bring pressure into the cylindrical hollow chamber, which interacts with the said braided sleeve: being the latter formed by inextensible threads, it can not follow in a homogeneous way the deformation undergone by the cylindrical hollow chamber, of elastic material.

From extensive studies, researches and experiments, it was found that the decisive factor to determine how the braided sleeve reacts, depends on the angle that each of the helical paths form with the longitudinal axis of said device when it is at rest; more precisely, it is seen that:
- at an angle of exactly 54.7° it only gets a stiffening of the device, or muscle, so without any changes in length and lateral deformations;
- with an angle smaller than 54.7° an axial contraction and an increase in the radial direction are obtained;
- with an angle greater than 54.7° an axial extension and a reduction in the radial direction are obtained.

At the end of the pressuring action, the device returns to its original position.

In another prior art document, US 5,165,323 A, is described a pneumatic actuator similar to a McKibben muscle, comprising an elastic tubular element, the ends of which are associated to two head members made of rigid material.

The elastic tubular element defines in its interior a chamber, while on the outside is wrapped in adhesion to a reinforcing mesh structure, consisting of threads of traction-resistant material, for example polyester, such as to be less yielding than the tubular elastic element.

The threads of the mesh, with the actuator at rest, have a predetermined angle of inclination with respect to the longitudinal axis of the actuator itself, which can be smaller than or greater than a characteristic angle, so-called of 'stand-by', that may have the previously indicated value.

Through a hole made in one of said head members, it is possible to pressurize the inner chamber; also in this case you get an approach or a withdrawal of the head members, that means a contraction or an extension of the actuator, in function of the angle of inclination originally established for the threads of the mesh, similarly to what previously said.

In the mentioned document it is claimed the presence of two sleeves which extend from the head members inside the chamber and telescopically mate, in order to guide the axial movement of the actuator.

Among the sleeves are shown sealing rings (O-rings) which isolate the chamber from surrounding possible losses; the volume of the chamber is reduced by the presence of the sleeves and requires a smaller air flow to be pressurized.

The drawback that most complains in the prior art devices classifiable as McKibben muscles concerns the fact that the pneumatic muscle response characteristics are laid down originally in function of the predetermined angle for the threads with respect to the longitudinal axis.

This means that a pneumatic muscle built for contraction will not stretch and vice versa, as well as a muscle that provides the angle exactly at 54.7° can not do other than stiffen without changing its extension.

In certain applications, where it is necessary to handle a generic user in opposite directions, therefore, we must combine two of said devices, with understandable constructive complications as well as increase of dimensions and costs.

Another drawback of the known devices concerns the fact that the predetermined angle of inclination not only determines the type of response, but also the extension or contraction stroke length, which can not be changed retrospectively.

Some researchers have experimented a technical solution to obtain actuators that could produce bi-directional movements: such solution provides an auxiliary chamber inside of the main one, adapted to be pressurized independently.

When the auxiliary chamber is not pressurized, the actuator is arranged for a shrinking movement as result of the pressurization of the main chamber; the volume occupied by the auxiliary chamber decreases request of air for the main chamber giving greater strength to the action.

When only the auxiliary chamber is pressurized, it expands axially causing a corresponding lengthening of the device.

The main drawback of this solution concerns the fact that the force exerted by the device is different in the two working conditions, with the one in extension considerably smaller than that in a contraction.

Additionally two power circuits are needed, or at least exchange valves to divert the flow of compressed air towards one or the other chamber.

All this results, inevitably, in an increase of the overall dimensions and weights.

Finally, also the limitation of other solutions in which the characteristics are predefined and not editable remains.

### Summary of the invention

The object of the present invention is therefore to propose a pneumatic device for the actuation of organs, which is similar to the working principles of McKibben type artificial muscles and similar, but shaped so as to obviate the limits of the known embodiments, in particular as regards the ability to be configured at will at any time, to obtain an active phase in the contraction, or in extension or only stiffening.

An another object of the invention resides in the desire to provide a device that offers the above mentioned characteristics by means of constructive solutions at the same time simple, effective and reliable.

Still another object of the invention aims to obtain a device of compact size and regular shape, so as to be easily installed in all applications without special care.

A further object of the invention is to propose a device which can be built in series with contained costs, by using materials, machineries and equipments of common use in the industry.

These and other objects are fully achieved by a pneumatic device for the actuation of organs, in which are provided: a cylindrical hollow chamber, tight, made of elastomeric material, adapted to be pressurized with air or other fluid; a braided sleeve, coaxially disposed outside said hollow cylindrical chamber and constituted by a first set of flexible and substantially inextensible wires, arranged each one according to its own helical path of the same diameter and helix angle but different principles, and by a second set of the same flexible and substantially inextensible wires, arranged according to respective helical paths simmetric to those of the above mentioned first set, with said first and second set of flexible threads adapted to form the meshes of said net, in which each of said helical paths is inclined of a same predetermined angle with respect to the longitudinal axis of above said device; two rigid head members, provided to the corresponding ends of said braided sleeve, to which are fixed the respective ends of the mentioned wires, with the same rigid head members provided to be mechanically connected to external users means; blow in-organs, adpted to pressurize or depressurize said cylindrical hollow chamber, respectively in an active phase, or in a resting phase of the said device, in the first of which said hollow cylindrical chamber interacts with the above mentioned braided sleeve to get an extension, or a contraction, or a stiffening of the same device, respectively, if the mentioned angle of inclination of each helical path is, originally, greater than, smaller than or equal to a predetermined value, with said device comprising: adjusting means, associated with said rigid head members, adpted to modify, increasing or decreasing, the distance originally provided between the same rigid head members in said stand-by phase of the device, so as to require a proportional and coherent variation of the angle of inclination of each helical path in said braided sleeve, such that said device, brought in its mentioned active phase, switches its response characteristics, determining for itself an axial extension, or an axial contraction, or a stiffening, respectively if, with a given distance in stand-by between the said rigid head members, the said angle of inclination is greater than, smaller than or equal to said predetermined value.

### Description Of Drawings

The characteristics of the invention will be made evident in the following description of preferred embodiments of the pneumatic device for the actuation of organs in the object, in accordance with the contents of the claims and with the help of the enclosed drawings, in which:
- Fig. 1 shows a first embodiment of the device in object;
- Fig. 2 shows an axial section of the device of Fig. 1;
- Fig. 3 shows an axial section of a second embodiment of the device;
- Fig. 4 shows an axial section of a third embodiment of the device;
- Figs. 5A, 5B, 5C schematically show an embodiment arranged with two devices parallel matched, having the same initial adjustment, in different operative phases;
- Figs. 6A, 6B schematically show an embodiment arranged similar to the previous one with the two devices having different initial adjustment, in two operating steps;
- Figs. 7A, 7B schematically show an embodiment arranged similar to the previous one with the two devices yet having identical initial adjustment, opposite to that of Fig. 5A, in two operating steps;
- Figs. 8A, 8B schematically show an embodiment in which the device is arranged to move a control lever in two opposite directions;
- Fig. 9 show the device applied to an orthopedic device;
- Fig. 10 schematically show an arrangement that provides multiple devices adapted to interact to obtain complex movements.

### Detailed description of preferred embodiments

In the above listed figures, it has been indicated with reference 1, the pneumatic device for the actuating organs, object of the present invention.

The device 1 follows the general principles of operation of the McKibben type artificial muscles and similar, of which it is said in the introduction, and provides, in an itself known manner:
- a hollow cylindrical chamber 2, tight, made of elastomeric material, adapted to be pressurized with air or other fluid by external means of blowing, not illustrated, constituted, for example, by a compressed air supply system;
- a braided sleeve 3, coaxially arranged outside said hollow cylindrical chamber 2 and constituted of a first set of threads 31 flexible and substantially inextensible, arranged each according to its own helical path of the same diameter and helix angle β but different principles, and by a second set of identical threads 32 flexible and substantially inextensible, arranged according to respective helical paths symmetrical to those of the above mentioned first set, with said first and second set of flexible threads 31, 32 adapted to form the meshes of said net 3, in which each of said helical paths is inclined of a same predetermined angle β with respect to the longitudinal axis X of the above said device 1;
- two rigid head members 4A, 4B provided at the corresponding ends of said braided sleeve 3, to which are fixed the respective ends of the mentioned threads 31, 32, with the same rigid head members 4A, 4B provided to be mechanically connected to external users means, not illustrated.

The blow-in bodies are adapted to pressurize or depressurize said hollow cylindrical chamber 2, respectively in an active phase K or in a stand-by phase W of the mentioned device 1, in the first of which said hollow cylindrical chamber 2 interacts with the said braided sleeve 3 to obtain an extension or a contraction, or a stiffening of the same device 1, respectively, if the mentioned angle of inclination b of each helical path is, originally, greater than, less than or equal to a predetermined value.The above mentioned predetermined value corresponds to an angle of 54.7° which is a theoretical limit that has been verified and experimentally validated.

According to the invention, the device 1 comprises adjusting means 5, associated to said rigid head members 4A, 4B and adapted to modify, increasing or decreasing, the distance D originally provided between the same rigid head members 4A, 4B in said phase of stand-by W of the device 1, so as to require a proportional and coherent variation of the angle of inclination β of each helical path in the above said braided sleeve 3.

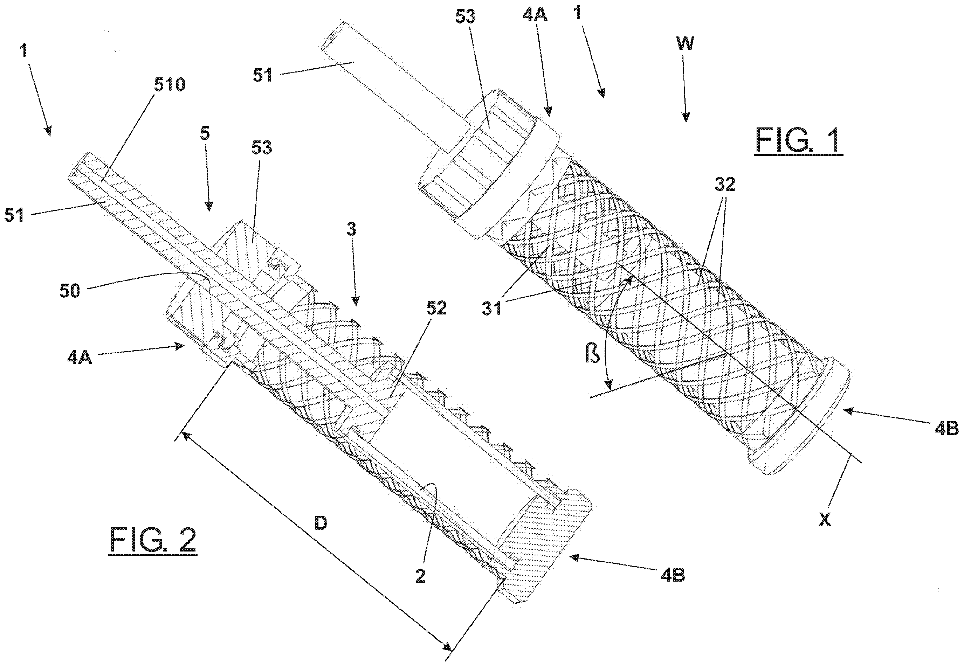

In a first embodiment of the device 1, illustrated in Figs. 1 and 2, said adjusting means 5 comprise:
- an axial through hole 50 made in one of said rigid head members, for example the head 4A;
- a stem 51, inserted into the axial through hole 50 and extended on both sides with respect to the mentioned rigid head 4A;
- a end plate 52, made integral to the end of the stem 51 located in an intermediate position between said rigid head members 4A, 4B inside said braided sleeve 3, said end plate 52 being placed in closure of a corresponding end of said hollow cylindrical chamber 2;
- operating means 53, interposed between the axial through hole 50 and the stem 51, adapted to allow axial translations of the respective rigid head 4A with respect to the stem 51 itself, thereby increasing or decreasing the distance D with the remaining rigid head 4B and the length of the braided sleeve 3.

In a preferred constructional solution, said axial through hole 50 and the stem 51 are threaded and mutually engaged, and the mentioned operating means 53 are constituted by a rotating pawl provided in said rigid head 4A and bearing the axial threaded through hole 50.

The pawl 53 can be rotated in one direction or the other to determine the above said offsets of the corresponding rigid head 4A along the stem 51; the position reached from time to time is automatically stabilized by the above mentioned threaded coupling between the through hole 50 and the stem 51.

In the embodiment referred to in Figs. 1 and 2, the stem 51 is provided with an axial passing through conduit 510, adapted to connect the said blowing means with the hollow cylindrical chamber 2; alternatively it is possible to provide a passing through duct made in the remaining rigid head 4B.

With the rotation of the pawl 53, and the consequent variation of the distance D between the rigid head members 4A, 4B, is determined the initial value of the said angle of inclination β of each helical path, so that the device 1, brought in its mentioned active stage K, switches its response characteristics, according to the following correlations:
- with an angle β of exactly 54.7° it only gets a stiffening of the device 1 so without changes in length of the same and without lateral deformations;
- with an angle β1 less than 54.7°, imposed increasing the distance D, it gets an axial contraction and an increase in the radial direction;
- with an angle β2 greater than 54.7°, imposed by decreasing the distance D, it gets an axial extension and a reduction in the radial direction.

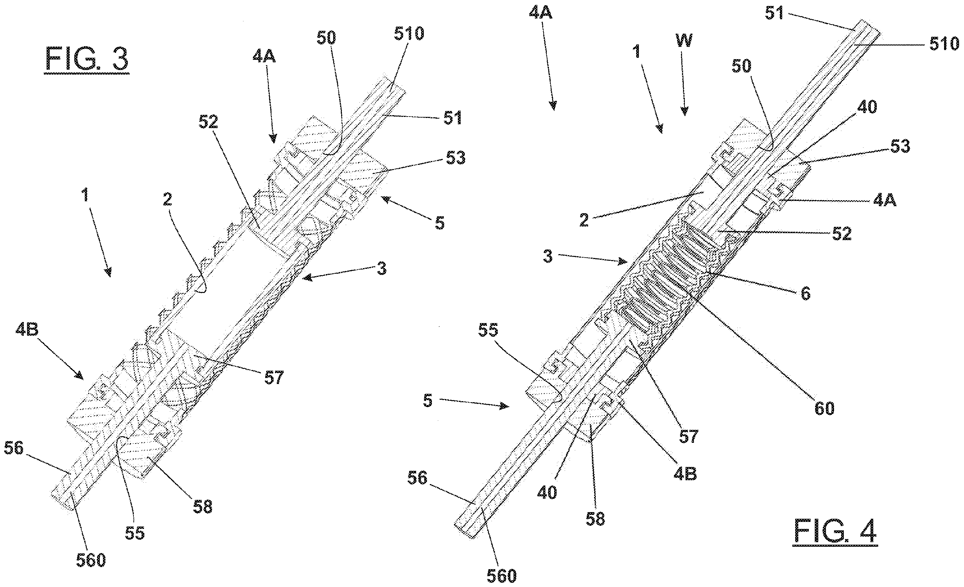

In a second embodiment of the device 1, illustrated in Fig. 3, said adjusting means 5 comprise:
- a first and a second axial through hole 50, 55 respectively, made in said rigid head members 4A, 4B;
- a first and a second stem 51, 56, respectively inserted into said first and second axial through hole 50, 55, with each of said stem 51, 56 extending both sides with respect to the corresponding rigid head 4A, 4B;
- a first and a second end plate 52, 57, each of which is made integral with the end of the respective stem 51, 56 located in an intermediate position between the rigid head members 4A, 4B inside the braided sleeve 3, said first and second end plate 52, 57 being in closing position of the corresponding ends of said hollow cylindrical chamber 2, situated in an intermediate position with respect to the braided sleeve 3;
- first and second operating means 53, 58, interposed respectively between the said first hole 50 and first stem 51 and between the said second hole 55 and second stem 56, adapted to allow axial translations of the respective rigid head 4A, 4B with respect to the corresponding stem 51, 56, thereby increasing or decreasing the distance D with the remaining rigid head 4A, 4B, as well as the length of said braided sleeve 3.

In a preferred constructive solution, in compliance with what already provided for said first form of embodiment, the first axial through hole 50 and the first stem 51 are threaded and mutually engaged, as well as the second axial through hole 55 and the second stem 56.

The first operating means 53 are constituted by a first rotatable pawl provided in the relative rigid head 4A and bearing the first threaded axial through hole 50; similarly, the second operating means 58 are constituted by a second rotatable pawl, for example, equal to the first, provided for in the remaining rigid head 4b and bearing the second threaded axial through hole 55.

Each pawl 53, 58 can be rotated in one direction or the other, independently, to determine axial translations of the corresponding rigid head 4A, 4B with respect to the other.

The threaded couplings between the through holes 50, 55 and respective stems 51, 56 stabilize the position which is time after time achieved by each rigid head 4A, 4B.

In the embodiment of Fig. 3, both stems 51, 56 are provided with an axial through conduit 510, 560, each adapted to connect the said blowing means with the hollow cylindrical chamber 2; alternatively it is possible to provide only one passing through duct in one of the two stems 51, 56.

As already explained with regard to the first embodiment, with the rotation of the first and / or of the second pawl 53, 58 and the consequent variation of the distance D between the rigid head members 4A, 4B, it is determined the initial value of the mentioned inclination angle β of each helical path, so that the device 1, taken in its mentioned active phase K, switches its response characteristics, according to the correlations previously set out.

The described second embodiment of the device 1 extends the calibration interval granted to said adjusting means 5, thereby increasing the versatility of the device 1 itself.

In a third embodiment of the device 1, illustrated in Fig. 4, said hollow cylindrical chamber 2 is associated fitting inside said braided sleeve 3 and has the same axial development of this.

As a constructional variant, it is possible to predict that the hollow cylindrical chamber 2 is incorporated in the sleeve 3 itself, though remaining the structural differences of the one and the other which make the first one elastic and the second one formed by substantially inextensible wires.

The corresponding adjusting means 5 comprise:
- a first and a second axial through hole 50, 55, respectively made in said rigid head members 4A, 4B;
- a first and a second stem 51, 56, respectively inserted into said first and second axial through hole 50, 55, with each of said stems 51, 56 extending both sides with respect to the corresponding rigid head 4A, 4B;
- a first and a second end plate 52, 57, each of which is made integral with the end of the respective stem 51, 56 located in an intermediate position between the rigid head members 4A, 4B inside the braided sleeve 3 and the hollow cylindrical chamber 2, said first and second end plate 52, 57 being connected to the opposite ends of a bellows 6 of elastic material, located in an intermediate position with respect to said braided sleeve 3 and hollow cylindrical chamber 2;
- at least one elastic member 60, housed in said bellows 6 and interposed between the said bottoms 52, 57, adapted to resiliently push the same in mutual removal;
- first and second operating members 53, 58, interposed respectively between the said first hole 50 and first stem 51 and between the said second hole 55 and second stem 56, adapted to allow axial translations of the respective rigid head 4A, 4B with respect to the corresponding stem 51, 56, thereby increasing or decreasing the distance D with the remaining rigid head 4A, 4B, as well as the length of said braided sleeve 3.

In a preferred constructive solution, in compliance with what already provided for the previous forms of embodiment, the first axial through hole 50 and the first stem 51 are threaded and mutually engaged, as well as the second axial through hole 55 and the second stem 56.

The respective first and second operating members 53, 58 are similarly constituted by a first and a second rotatable pawl provided in the relative rigid head members 4A, 4B adapted to be rotated in one direction or another, in an independent manner, to determine such axial offsets of the corresponding rigid head 4A, 4B with respect to the other.

Also in this case, the threaded couplings between the through holes 50, 55 and the respective stems 51, 56 stabilize the position reached time after time by each rigid head 4A, 4B.

The elastic member 60 inside the bellows 6 stabilizes the structure of the device 1 during the calibration operations of the angle of inclination β.

In the embodiment of Fig. 4, both stems 51, 56 are provided with an axial through conduit 510, 560, each adapted to connect the said blowing means with the interior of the bellows 6, which in turn has openings (not shown in detail) which allow the introduced pressurized air flow to merge inside the hollow cylindrical chamber 2; alternatively it is possible to provide only one passing through duct in one of the two stems 51,56.

In each of the rigid head members 4A, 4B is provided a tight o-ring 40 which engages the respective stem 51, 56 and prevents the compressed air to escape from the hollow cylindrical chamber 2 through the through holes 50, 55.

During the active step K of the device 1, the bellows 6 as well as the elastic member 60 are compressed or elongated in agreement with the contraction or extension of the device 1 itself.

In the figures from 5A to 7B is illustrated schematically an application with two devices 1 matched parallel, mechanically connected to a fixed upper support 70 and to a lower bar 80.

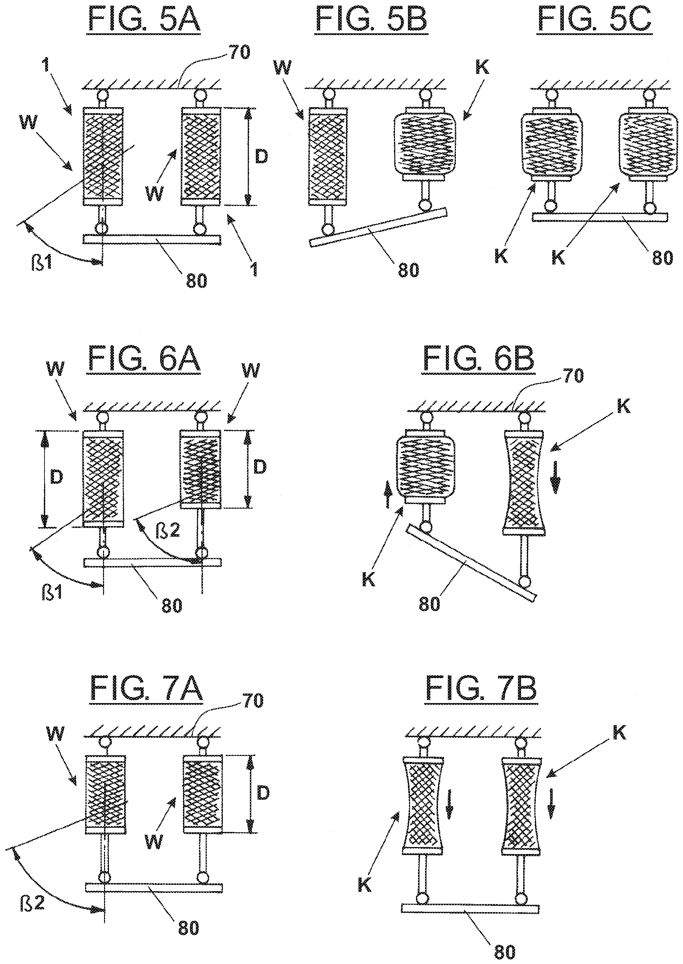

In Fig. 5A the devices 1 have the same initial adjustment, with an angle of inclination β1 of the helical paths of 54.7°, imposed by increasing the distance D between the rigid head members 4A, 4B; both the devices 1 are then prepared for an axial contraction and an increase in the radial direction.

In Fig. 5B only the device 1 on the right is in its active phase K, for which the bar 80 is sloped, while with the activation of both devices 1, as shown in Fig. 5C, the bar 80 is again horizontal but at a level higher than that one of departure.

In Fig. 6A the devices 1 have different initial adjustment: the left one presents an angle of inclination of the helical paths β1 smaller than 54.7°, therefore predisposed to the contraction, while the right one has an angle β2 greater than 54,7°, then predisposed to the extension.

Fig. 6B shows how the bar 80 is sloped (with higher angle with respect to the position taken in Fig. 5B) with the simultaneous activation of the two devices 1.

In Fig. 7A the devices 1 have the same initial adjustment, with an angle β2 greater than 54.7°, then both are predisposed to the extension, as shown in Fig. 7B, in which the bar 80 moves parallel to the initial position and places itself at a lower level.

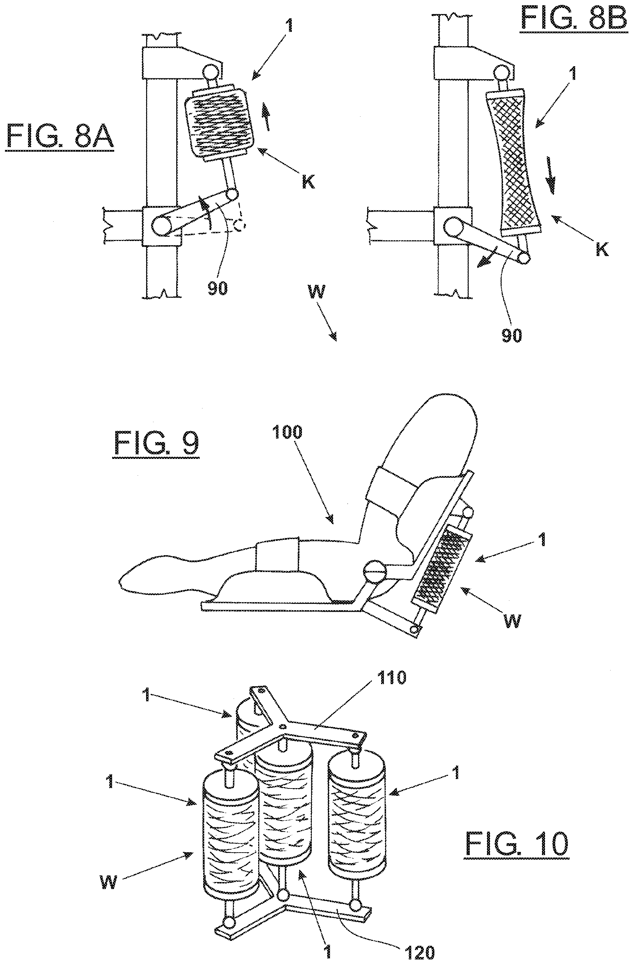

In Figs. 8A, 8B is illustrated schematically an application in which the device 1 is set up to move a control bar 90, for example associated with a flow diverter valve.

As it can be seen, depending on the initial adjustment of the angle β the bar 90 can be driven in two opposite directions.

In Fig. 9 is illustrated, again as a way of example, another possible application in which the device 1 is associated with an orthopedic device 100 for the rehabilitation of an upper limb; according to the usual adjustment of the angle β it will be possible to get the desired bending movement.

In Fig. 10 is illustrated a further possible application that provides the presence of four devices 1, parallel to each other and radially arranged.

The devices 1 are connected at the top to a first supporting cross 110, and at the bottom to a second supporting cross 120, for example equal to the first.

Depending on how the angles β of each device 1 are adjusted, and on the activation mode of these, it can be obtained different orientations between said supporting crosses 110, 120, similar to those granted by a ball joint, but with the application of thrust forces; it understandable how similar applications are interesting in the field of robotics and / or bio-robotics.

From the previous description emerge in the evidence the peculiar characteristics of the proposed device, which incorporates the operating principles of McKibben type artificial muscle and similar, but it introduces original construction solutions that allow it to be configured at will at any time, to obtain an active phase in the contraction, or in extension or only of stiffening.

With this important prerogative they are totally exceeded the limits of the known embodiments and it is greatly simplified any practical application of the device.

It should be highlighted how the advantageous qualities of the proposed device are obtained with techniques at the same time simple, effective and reliable which allow besides to have a compact size and regular shape.

All the constructional solutions adopted meet the requirements that allow an easy serial production of the device with contained costs, as it could be used materials, machinery and equipment of common use in the industry.

As intuitively understandable, the device can be employed in various sectors of industry, automation, robotics, bio-medical, just to name a few.

It is understood however that what above said has value of example and not limiting, therefore any modifications of detail that may be necessary to make for technical and / or functional reasons, are considered from now as remaining within the protective scope defined by the claims below.

## Claims

1. Pneumatic actuating device for the actuation of organs, providing: an airtight hollow cylindrical chamber (2), made of elastomeric material, adapted to be pressurized with air or other fluid; a braided sleeve (3), disposed coaxially outside said hollow cylindrical chamber (2) and constituted by a first series of flexible and substantially inextensible threads (31), arranged each according to its own helical path of the same diameter and helix angle (β) but different start points, and by a second series of identical flexible and substantially inextensible threads (32), arranged according to respective helical paths symmetrical to those of the above mentioned first series, with said first and second set of flexible threads (31, 32) adapted to form the meshes of said braided sleeve (3), in which each of said helical paths is inclined with a same predetermined angle (β) with respect to the longitudinal axis (X) of said device (1); two rigid head members (4A, 4B), provided at the corresponding ends of said braided sleeve (3), to which are fixed at the respective start points of the mentioned threads (31, 32), with the same rigid head members (4A, 4B) adapted to be mechanically connected to external utilizing means; blowing means, adapted to pressurize or depressurize said hollow cylindrical chamber (2), respectively in an active phase (K) or in a stand-by phase (W) of said device (1), in the first of which said hollow cylindrical chamber (2) interacts with the said braided sleeve (3) to obtain an extension or a contraction, or a stiffening of the same device (1), respectively, if the aforementioned angle of inclination (β) of each helical path is, originally, greater than, less than or equal to a predetermined value, with said device (1) **characterized in that** it comprises: adjusting means (5), associated to said rigid head members (4A, 4B), adapted to modify, by increasing or decreasing, the distance (D) originally provided between the same rigid head members (4A, 4B) in said stand-by phase (W) of the device (1), so as to cause a proportional and coherent variation of the angle of inclination (β) of each helix in said braided sleeve (3), such that the response of said device (1) when it is pressurized in its mentioned active phase (K), switches with determining an axial extension, or an axial contraction, or a stiffening, respectively, if, with a given distance (D) in the stand-by phase between said rigid head members (4A, 4B), said angle of inclination (β) is greater than, less than or equal to said predetermined value, and wherein said predetermined value is 54.7°.

2. Device according to claim 1, **characterized in that** said adjusting means (5) comprise: an axial through hole (50) realized in one of said rigid head members (4A); a stem (51), inserted in said axial through hole (50) extended form both sides of said rigid head member (4A); a end plate (52), made integral with the end of the said stem (51) located in an intermediate position between said rigid head members (4A, 4B), inside said braided sleeve (3), said end plate (52) being placed to close a corresponding end of said hollow cylindrical chamber (2); operating means (53), interposed between said axial through hole (50) and the stem (51), adapted to allow axial offsets of the mentioned rigid head member (4A) with respect to the stem (51) itself, thereby increasing or decreasing the distance (D) with the remaining rigid head member (4B), as well as the length of the above braided sleeve (3), with the same operating means (53) also adapted to lock the position set for said rigid head member (4A).

3. Device according to claim 1, **characterized in that** said adjusting means (5) comprise: a first and a second axial through hole (50, 55) respectively made in said rigid head members (4A, 4B); a first and a second stem (51, 56), respectively inserted in said first and second axial through holes (50, 55), with each of said stems (51, 56) extending boths sides from the corresponding rigid head member (4A, 4B); a first and a second end plate (52, 57), each of which made integral with the end of the respective stem (51, 56) located in an intermediate position between said rigid head members (4A, 4B), inside said braided sleeve (3), said first and second end plate (52, 57) being arranged for closing the corresponding ends of said hollow cylindrical chamber (2), placed in an intermediate position with respect to said braided sleeve (3); first and second operating members (53, 58), interposed respectively between the said first axial through hole (50) and first stem (51) and between the said second axial through hole (55) and second stem (56), adapted to allow axial offsets of the respective rigid head member (4A, 4B) compared to the corresponding stem (51, 56), thereby increasing or decreasing the distance (D) with the remaining rigid head member (4A, 4B), as well as the length of the above braided sleeve (3), with the same first and second operating members (53, 58) also adapted to lock the position set for the corresponding rigid head member (4A, 4B).

4. Device according to claim 1, **characterized in that** said hollow cylindrical chamber (2) fits inside said braided sleeve (3) and it has the same axial extension, and **in that** said adjusting means (5) comprises: a first and a second axial through hole (50, 55) respectively made in said rigid head members (4A, 4B); a first and a second stems (51, 56), respectively inserted in said first and second axial through holes (50, 55), with each of said stems (51, 56) extending both sides from the corresponding rigid head member (4A, 4B) ; a first and a second end plates (52, 57), each of which is made integral with the end of the respective stem (51, 56) located in an intermediate position between said rigid head members (4A, 4B), inside said braided sleeve (3) and hollow cylindrical chamber (2), said first and second end plates (52, 57) being connected to the opposite ends of a bellows (6) of resilient material, located in an intermediate position with respect to said braided sleeve (3) and hollow cylindrical chamber (2); at least one elastic member (60), housed in said bellows (6) and interposed between the said end plates (52, 57), adapted to elastically push away one from the other said end plates; first and second operating members (53, 58), respectively interposed between the said first axial through hole (50) and first stem (51) and between the said second axial through hole (55) and second stem (56), adapted to allow axial offsets of the respective rigid head member (4A, 4B) compared to the corresponding stem (51, 56), thereby increasing or decreasing the distance (D) with the remaining rigid head member (4A, 4B), as well as the length of the above braided sleeve (3), with the same first and second operating members (53, 58) also adapted to lock the position set for the corresponding rigid head member (4A, 4B).

5. Device according to claim 2, **characterized in that** said axial through hole (50) and stem (51) are threaded and mutually engaged, **by the fact that** said operating means (53, 58) are constituted by a rotatable pawl provided in the said rigid head member (4A) and bearing the mentioned threaded first axial through hole (50), with said pawl (53) adapted to be rotated in one direction or in the other to determine the above mentioned axial translations of the rigid head member (4A) along the stem (51), **and in that** said threaded coupling between said first axial through hole (50) and stem (51) is adapted to stabilize the position time after time reached by the same rigid head member (4A).

6. Device according to claim 2 or 5, **characterized in that** said stem (51) is provided with an axial through conduit (510), adapted to connect blowing means with said hollow cylindrical chamber (2), said blowing means being provided outside said device (1) and adapted to pressurize or depressurize the latter.

7. Device according to claim 3 or 4, **characterized in that** said first and second axial through hole (50, 55) and first and second stem (51, 56) are threaded and mutually engaged, **by the fact that** the said operating means (53, 58) consists of a first and a second rotatable pawl provided in the relative rigid head members (4A, 4B) and bearing said first and second threaded axial through hole (50, 55), with said first and second pawl (53, 58) adapted to be rotated in a direction or the other, in an independent manner, to determine the above mentioned axial offsets of the corresponding rigid head member (4A, 4B) relative to each other along the stems (51, 56), and **in that** said threaded couplings between said first and second axial through holes (50, 55) and relative stems (51, 56) are adapted to stabilize the position which is reached time after time by each rigid head member (4A, 4B).

8. Device according to claim 3, **characterized in that** at least one of said stems (51, 56) is provided with a axial through conduit (510, 560), adapted to connect blowing means with said hollow cylindrical chamber (2), the said blowing means being provided on the outside of the mentioned device (1) and adapted to pressurize or depressurize the latter.

9. Device according to claim 4, **characterized in that** at least one of said stems (51, 56) is provided with a axial through conduit (510, 560), adapted to connect blowing means, provided outside of said device (1) to send a fluid under pressure, with the interior of said bellows (6), which in turn is affected by openings for the passage of air under pressure towards the interior of said hollow cylindrical chamber (2) intended to be pressurized.

10. Device according to claim 4, **characterized in that** said hollow cylindrical chamber (2) is embedded in said braided sleeve (3), with the first being elastic and with the second defined by substantially inextensible threads (31,32).

## Patentansprüche

1. Pneumatische Betätigungsvorrichtung für die Betätigung von Organen, die Folgendes umfasst: eine luftdichte hohle zylindrische Kammer (2) aus elastomeren Material, die mit Luft oder einem anderen Fluid unter Druck gesetzt werden kann; eine geflochtene Hülse (3), die koaxial außerhalb der hohlen zylindrischen Kammer (2) angeordnet ist und aus einer ersten Reihe von flexiblen und im wesentlichen nicht dehnbaren Fäden (31) besteht, die jeweils gemäß ihrer eigenen spiralförmigen Bahn mit gleichem Durchmesser und gleichem Spiralwinkel (β), aber unterschiedlichen Anfangspunkten angeordnet sind, sowie aus einer zweiten Reihe von identischen flexiblen und im wesentlichen nicht dehnbaren Fäden (32), die entsprechend jeweiligen schraubenförmigen Bahnen angeordnet sind, die symmetrisch zu denen der oben erwähnten ersten Reihe sind, wobei der erste und der zweite Satz flexibler Fäden (31, 32) geeignet ist, die Maschen der geflochtenen Hülse (3) zu bilden, wobei jede der schraubenförmigen Bahnen mit einem gleichen vorbestimmten Winkel (β) in Bezug auf die Längsachse (X) der Vorrichtung (1) geneigt ist; zwei starre Kopfelemente (4A, 4B), die an den entsprechenden Enden der geflochtenen Hülse (3) vorgesehen sind, an denen an den jeweiligen Startpunkten der erwähnten Fäden (31, 32) befestigt sind, wobei dieselben starren Kopfelemente (4A, 4B) mechanisch mit externen Verwendungsmitteln verbunden werden können; Blasmittel, die geeignet sind, die hohle zylindrische Kammer (2) in einer aktiven Phase (K) bzw. in einer Bereitschaftsphase (W) der Vorrichtung (1) unter Druck zu setzen oder zu entlasten, wobei in der ersten davon die hohle zylindrische Kammer (2) mit der geflochtenen Hülse (3) zusammenwirkt, um eine Ausdehnung oder eine Kontraktion zu erhalten, bzw. eine Versteifung derselben Vorrichtung (1), wenn der vorgenannte Neigungswinkel (β) jeder schraubenförmigen Bahn ursprünglich größer, kleiner oder gleich einem vorbestimmten Wert ist, wobei die Vorrichtung (1) **dadurch gekennzeichnet ist, dass** sie umfasst: Einstellmittel (5), die den starren Kopfelementen (4A, 4B) zugeordnet und geeignet sind, durch Vergrößern oder Verkleinern des ursprünglich zwischen denselben starren Kopfelementen (4A, 4B) in der Bereitschaftsphase (W) der Vorrichtung (1) vorgesehenen Abstands (D) zu modifizieren, um eine proportionale und kohärente Veränderung des Neigungswinkels (β) jeder Wendel in der geflochtenen Hülse (3) zu bewirken, derart, daß das Ansprechen der Vorrichtung (1), wenn sie in ihrer erwähnten aktiven Phase (K) unter Druck gesetzt wird, mit der Bestimmung einer axialen Ausdehnung oder einer axialen Kontraktion bzw. einer Versteifung schaltet, wenn mit einem gegebenen Abstand (D) in der Bereitschaftsphase zwischen den starren Kopfteilen (4A, 4B) der Neigungswinkel (β) größer, kleiner oder gleich dem vorbestimmten Wert ist, und wobei der vorbestimmte Wert 54,7° beträgt.

2. Vorrichtung nach Anspruch 1, **dadurch gekennzeichnet, dass** die Einstellmittel (5) umfassen: ein axiales Durchgangsloch (50), das in einem der starren Kopfelemente (4A) ausgebildet ist; einen Schaft (51), der in das axiale Durchgangsloch (50) eingeführt ist und sich von beiden Seiten des starren Kopfelements (4A) erstreckt; eine Endplatte (52), die einstückig mit dem Ende des Schafts (51) ausgebildet ist, das sich in einer Zwischenposition zwischen den starren Kopfelementen (4A, 4B) innerhalb der geflochtenen Hülse (3) befindet, wobei die Endplatte (52) so angeordnet ist, dass sie ein entsprechendes Ende der hohlen zylindrischen Kammer (2) verschließt; Betätigungsmittel (53), die zwischen dem axialen Durchgangsloch (50) und dem Schaft (51) angeordnet und so ausgebildet sind, dass sie axiale Versetzungen des erwähnten starren Kopfteils (4A) in Bezug auf den Schaft (51) selbst zulassen, wodurch der Abstand (D) mit dem verbleibenden starren Kopfteil (4B) sowie die Länge der oben genannten geflochtenen Hülse (3) vergrößert oder verringert wird, wobei die gleichen Betätigungsmittel (53) auch so ausgebildet sind, dass sie die für das starre Kopfteil (4A) eingestellte Position verriegeln.

3. Vorrichtung nach Anspruch 1, **dadurch gekennzeichnet, dass** die Einstellmittel (5) umfassen: ein erstes und ein zweites axiales Durchgangsloch (50, 55), die jeweils in den starren Kopfelementen (4 A, 4B) ausgebildet sind; einen ersten und einen zweiten Schaft (51, 56), die jeweils in das erste und das zweite axiale Durchgangsloch (50, 55) eingesetzt sind, wobei sich jeder der Schäfte (51, 56) beidseitig von dem entsprechenden starren Kopfelement (4 A, 4B) erstreckt; eine erste und eine zweite Endplatte (52, 57), von denen jede einstückig mit dem Ende des jeweiligen Schaftes (51, 56) ausgebildet ist, der sich in einer Zwischenposition zwischen den starren Kopfelementen (4A, 4B) innerhalb der geflochtenen Hülse (3) befindet, wobei die erste und die zweite Endplatte (52, 57) zum Verschließen der entsprechenden Enden der hohlen zylindrischen Kammer (2) angeordnet sind, die sich in einer Zwischenposition in Bezug auf die geflochtene Hülse (3) befindet; ein erstes und ein zweites Betätigungselement (53, 58), die zwischen dem ersten axialen Durchgangsloch (50) und dem ersten Schaft (51) bzw. zwischen dem zweiten axialen Durchgangsloch (55) und dem zweiten Schaft (56) angeordnet sind und so ausgebildet sind, dass sie eine axiale Verschiebung des jeweiligen starren Kopfelements (4A, 4B) gegenüber dem entsprechenden Schaft (51, 56) ermöglichen, wodurch sich der Abstand (D) mit dem verbleibenden starren Kopfelement (4A, 4B) sowie die Länge der obigen geflochtenen Hülse (3) vergrößert oder verkleinert, wobei die gleichen ersten und zweiten Betätigungselemente (53, 58) auch dazu geeignet sind, die für das entsprechenden starren Kopfelement (4A, 4B) eingestellte Position zu verriegeln.

4. Vorrichtung nach Anspruch 1, **dadurch gekennzeichnet, dass** die genannte hohle zylindrische Kammer (2) in die genannte geflochtene Hülse (3) passt und die gleiche axiale Ausdehnung hat, und dass das genannte Einstellmittel (5) umfasst: ein erstes und ein zweites axiales Durchgangsloch (50, 55), die jeweils in den starren Kopfelementen (4A, 4B) ausgebildet sind; einen ersten und einen zweiten Schaft (51, 56), die jeweils in das erste und das zweite axiale Durchgangsloch (50, 55) eingesetzt sind, wobei sich jeder der Schäfte (51, 56) auf beiden Seiten von dem entsprechenden starren Kopfelement (4A, 4B) aus erstreckt; eine erste und eine zweite Endplatte (52, 57), von denen jede einstückig mit dem Ende des jeweiligen Schafts (51, 56) ausgebildet ist, der sich in einer Zwischenposition zwischen den starren Kopfelementen (4A, 4B) innerhalb der geflochtenen Hülse (3) und der hohlen zylindrischen Kammer (2) befindet, wobei die erste und die zweite Endplatte (52, 57) mit den gegenüberliegenden Enden eines Balgs (6) aus elastischem Material verbunden sind, der sich in einer Zwischenposition in Bezug auf die geflochtene Hülse (3) und die hohle zylindrische Kammer (2) befindet; mindestens ein elastisches Element (60), das in dem genannten Balg (6) untergebracht und zwischen den genannten Endplatten (52, 57) angeordnet ist und dazu geeignet ist, die genannten Endplatten elastisch voneinander wegzudrücken; ein erstes und ein zweites Betätigungselement (53, 58), die jeweils zwischen dem ersten axialen Durchgangsloch (50) und dem ersten Schaft (51) bzw. zwischen dem zweiten axialen Durchgangsloch (55) und dem zweiten Schaft (56) angeordnet und so ausgebildet sind, dass sie eine axiale Verschiebung des jeweiligen starren Kopfelements (4A, 4B) gegenüber dem entsprechenden Schaft (51, 56) ermöglichen, wodurch sich der Abstand (D) mit dem verbleibenden starren Kopfelement (4A, 4B) sowie die Länge der obigen geflochtene Hülse (3) vergrößert oder verkleinert, wobei die gleichen ersten und zweiten Betätigungselemente (53, 58) auch dazu geeignet sind, die für das entsprechenden starren Kopfelement (4A, 4B) eingestellte Position zu verriegeln.

5. Vorrichtung nach Anspruch 2, **dadurch gekennzeichnet, dass** das axiale Durchgangsloch (50) und der Schaft (51) mit einem Gewinde versehen sind und miteinander in Eingriff stehen, dadurch, dass die Betätigungselemente (53, 58) durch eine drehbare Sperrklinke gebildet werden, die in dem starren Kopfelement (4A) vorgesehen ist und das erwähnte axiale Gewindedurchgangsloch (50) trägt, wobei die Sperrklinke (53) in die eine oder andere Richtung gedreht werden kann, um die oben erwähnten axialen Verschiebungen des starren Kopfelements (4A) entlang des Schafts (51) zu bestimmen, und wobei die Gewindekupplung zwischen dem Durchgangsloch (50) und dem Schaft (51) die Position stabilisieren kann, die von demselben starren Kopfelement (4A) immer wieder erreicht wird.

6. Vorrichtung nach Anspruch 2 oder 5, **dadurch gekennzeichnet, dass** der Schaft (51) mit einer axialen Durchgangsleitung (510) versehen ist, die geeignet ist, Blasmittel mit der hohlen zylindrischen Kammer (2) zu verbinden, wobei die Blasmittel außerhalb der Vorrichtung (1) vorgesehen und geeignet sind, letztere unter Druck zu setzen oder drucklos zu machen.

7. Vorrichtung nach Anspruch 3 oder 4, **dadurch gekennzeichnet, dass** das erste und zweite axiale Durchgangsloch (50, 55) und der erste und zweite Schaft (51, 56) mit einem Gewinde versehen sind und miteinander in Eingriff stehen, dadurch, dass das Betätigungselement (53, 58) aus einer ersten und zweiten drehbaren Sperrklinke besteht, die in den entsprechenden starren Kopfelementen (4A, 4B) vorgesehen sind und das erste und zweite axiale Gewindedurchgangsloch (50, 55), mit der ersten und zweiten Sperrklinke (53, 58) tragen, die geeignet sind, in der einen oder anderen Richtung in unabhängiger Weise gedreht zu werden, um die oben erwähnten axialen Versätze des entsprechenden starren Kopfelements (4A, 4B) relativ zueinander entlang der Schäfte (51, 56) zu bestimmen, und dass die Gewindeverbindungen zwischen den ersten und zweiten axialen Durchgangslöchern (50, 55) und den entsprechenden Schäften (51, 56) geeignet sind, die Position zu stabilisieren, die von jedem starren Kopfelement (4A, 4B) immer wieder erreicht wird.

8. Vorrichtung nach Anspruch 3, **dadurch gekennzeichnet, dass** mindestens einer der Schäfte (51, 56) mit einer axialen Durchgangsleitung (510, 560) versehen ist, die geeignet ist, Blasmittel mit der hohlen zylindrischen Kammer (2) zu verbinden, wobei die Blasmittel an der Außenseite der genannten Vorrichtung (1) vorgesehen sind und geeignet sind, diese unter Druck zu setzen oder drucklos zu machen.

9. Vorrichtung nach Anspruch 4, **dadurch gekennzeichnet, dass** mindestens einer der Schäfte (51, 56) mit einer axialen Durchgangsleitung (510, 560) versehen ist, die geeignet ist, Blasmittel, die außerhalb der Vorrichtung (1) vorgesehen sind, um ein unter Druck stehendes Fluid mit dem Inneren des Balgs (6) zu verbinden, der seinerseits von Öffnungen für den Durchgang von unter Druck stehender Luft in das Innere der hohlen zylindrischen Kammer (2), die unter Druck gesetzt werden soll, beeinflusst wird.

10. Vorrichtung nach Anspruch 4, **dadurch gekennzeichnet, dass** die hohle zylindrische Kammer (2) in die geflochtene Hülse (3) eingebettet ist, wobei die erste elastisch ist und die zweite durch im Wesentlichen nicht dehnbare Fäden (31, 32) definiert ist.

## Revendications

1. Dispositif d'actionnement pneumatique pour activer des organes, comprennent: une chambre cylindrique creuse étanche à l'air (2), en matériau élastomère, adaptée pour être pressurisée avec de l'air ou un autre fluide; un manchon tressé (3), disposé coaxialement à l'extérieur de ladite chambre cylindrique creuse (2) et constitué par une première série de fils flexibles et sensiblement inextensibles (31), disposés chacun selon son propre trajet hélicoïdal de même diamètre et angle d'hélice (β) mais de points de départ différents, et par une seconde série de fils flexibles et sensiblement inextensibles identiques (32), disposés selon des trajets hélicoïdaux respectifs symétriques à ceux de la première série mentionnée ci-dessus, avec lesdits premier et deuxième ensembles de fils flexibles (31, 32) adaptés pour former les mailles dudit manchon tressé (3), dans lequel chacun desdits trajets hélicoïdaux est incliné d'un même angle prédéterminé (β) par rapport à l'axe longitudinal (X) dudit dispositif (1); deux éléments de tête rigides (4A, 4B), prévus aux extrémités correspondantes dudit manchon tressé (3), auxquels sont fixés les points de départ respectifs des fils mentionnés (31, 32), les mêmes éléments de tête rigides (4A, 4B) étant adaptés pour être reliés mécaniquement à des moyens d'utilisation externes; des moyens de soufflage, adaptés pour pressuriser ou dépressuriser ladite chambre cylindrique creuse (2), respectivement dans une phase active (K) ou dans une phase d'attente (W) dudit dispositif (1), dans la première desquelles ladite chambre cylindrique creuse (2) interagit avec ledit manchon tressé (3) pour obtenir une extension ou une contraction, ou un raidissement du même dispositif (1), respectivement, si l'angle d'inclinaison précité (β) de chaque trajectoire hélicoïdale est, à l'origine, supérieur, inférieur ou égal à une valeur prédéterminée, ledit dispositif étant **caractérisé en ce qu'**il comprend: des moyens de réglage (5), associés auxdits éléments de tête rigides (4A, 4B), adaptés pour modifier, par augmentation ou diminution, la distance (D) prévue à l'origine entre les mêmes éléments de tête rigides (4A, 4B) dans ladite phase d'attente (W) du dispositif (1), de manière à provoquer une variation proportionnelle et cohérente de l'angle d'inclinaison (β) de chaque hélice dans ledit manchon tressé (3), de sorte que la réponse dudit dispositif (1) lorsqu'il est mis sous pression dans sa phase active mentionnée (K), commute en déterminant une extension axiale, ou une contraction axiale, ou un raidissement, respectivement, si, avec une distance donnée (D) dans la phase d'attente entre lesdits éléments de tête rigides (4A, 4B), ledit angle d'inclinaison (β) est supérieur, inférieur ou égal à ladite valeur prédéterminée, et dans lequel ladite valeur prédéterminée est de 54,7°.

2. Dispositif selon la revendication 1, **caractérisé en ce que** lesdits moyens de réglage (5) comprennent: un trou traversant axial (50) réalisé dans l'un desdits éléments de tête rigides (4A); une tige (51), insérée dans ledit trou traversant axial (50) s'étendant des deux côtés dudit élément de tête rigide (4A); une plaque d'extrémité (52), réalisée d'un seul tenant avec l'extrémité de ladite tige (51) située dans une position intermédiaire entre lesdits éléments de tête rigides (4A, 4B), à l'intérieur dudit manchon tressé (3), ladite plaque d'extrémité (52) étant placée pour fermer une extrémité correspondante de ladite chambre cylindrique creuse (2); des moyens d'actionnement (53), interposés entre ledit trou traversant axial (50) et la tige (51), adaptés pour permettre des décalages axiaux de l'élément de tête rigide mentionné (4A) par rapport à la tige (51) elle-même, augmentant ou diminuant ainsi la distance (D) avec l'élément de tête rigide restant (4B), ainsi que la longueur du manchon tressé ci-dessus (3), avec les mêmes moyens d'actionnement (53) également adaptés pour verrouiller la position définie pour ledit élément de tête rigide (4A).

3. Dispositif selon la revendication 1, **caractérisé en ce que** lesdites moyens de réglage (5) comprennent: un premier et un deuxième trou axial traversant (50, 55) respectivement réalisés dans lesdits éléments de tête rigides (4 A, 4B); une première et une deuxième tige (51,56), respectivement insérées dans lesdits premier et deuxième trous traversants axiaux chacune desdites tiges (51, 56) s'étendant sur les deux côtés de l'élément de tête rigide correspondant (4 A, 4B); une première et une seconde plaque d'extrémité (52, 57), chacune d'entre elles étant rendue solidaire de l'extrémité de la tige respective (51, 56) située dans une position intermédiaire entre lesdits éléments de tête rigides (4A, 4B), à l'intérieur dudit manchon tressé (3), lesdites première et seconde plaques d'extrémité (52, 57) étant agencées pour fermer les extrémités correspondantes de ladite chambre cylindrique creuse (2), placée dans une position intermédiaire par rapport audit manchon tressé (3); des premier et second éléments d'actionnement (53, 58), interposés respectivement entre ledit premier trou axial traversant (50) et la première tige (51) et entre ledit second trou axial traversant (55) et la seconde tige (56), adaptés pour permettre des décalages axiaux de l'élément de tête rigide respectif (4A, 4B) par rapport à la tige correspondante (51, 56), augmentant ou diminuant ainsi la distance (D) avec l'élément de tête rigide restant (4A, 4B), ainsi que la longueur du manchon tressé (3) ci-dessus, avec les mêmes premier et deuxième éléments de commande (53, 58) également adaptés pour verrouiller la position définie pour l'élément de tête rigide correspondant (4A, 4B).

4. Dispositif selon la revendication 1, **caractérisé en ce que** ladite chambre cylindrique creuse (2) s'adapte à l'intérieur dudit manchon tressé (3) et elle a la même extension axiale, et **en ce que** ledit moyen de réglage (5) comprend : un premier et un deuxième trou traversant axial (50, 55) respectivement réalisés dans lesdits éléments de tête rigides (4A, 4B); une première et une deuxième tiges (51, 56), respectivement insérées dans lesdits premier et deuxième trous traversants axiaux (50, 55), chacune desdites tiges (51, 56) s'étendant des deux côtés de l'élément de tête rigide correspondant (4A, 4B); une première et une seconde plaques d'extrémité (52, 57), dont chacune est rendue solidaire de l'extrémité de la tige respective (51, 56) située dans une position intermédiaire entre lesdits éléments de tête rigides (4A, 4B), à l'intérieur dudit manchon tressé (3) et de ladite chambre cylindrique creuse (2), lesdites première et seconde plaques d'extrémité (52, 57) étant reliées aux extrémités opposées d'un soufflet (6) en matériau élastique, situé dans une position intermédiaire par rapport audit manchon tressé (3) et à ladite chambre cylindrique creuse (2); au moins un élément élastique (60), logé dans ledit soufflet (6) et interposé entre lesdites plaques d'extrémité (52, 57), adapté pour écarter élastiquement l'une de l'autre lesdites plaques d'extrémité; des premier et second éléments d'actionnement (53, 58), respectivement interposés entre ledit premier trou axial traversant (50) et la première tige (51) et entre ledit second trou axial traversant (55) et la seconde tige (56), adaptés pour permettre des décalages axiaux de l'élément de tête rigide respectif (4A, 4B) par rapport à la tige correspondante (51, 56), augmentant ou diminuant ainsi la distance (D) avec l'élément de tête rigide restant (4 A, 4B), ainsi que la longueur du manchon tressé (3) ci-dessus, avec les mêmes premier et deuxième éléments de commande (53, 58) également adaptés pour verrouiller la position définie pour l'élément de tête rigide correspondant (4A, 4B).

5. Dispositif selon la revendication 2, **caractérisé en ce que** ledit trou axial traversant (50) et la tige (51) sont filetés et s'engagent mutuellement, par le fait que lesdits moyens d'actionnement (53, 58) sont constitués par un cliquet rotatif prévu dans ledit élément de tête rigide (4A) et portant le trou axial fileté (50) mentionné, ledit cliquet (53) étant adapté pour être tourné dans un sens ou dans l'autre pour déterminer les translations axiales susmentionnées de l'élément de tête rigide (4A) le long de la tige (51), et **en ce que** ledit accouplement fileté entre le trou traversant (50) et la tige (51) est adapté pour stabiliser la position atteinte à chaque fois par le même élément de tête rigide (4A).

6. Dispositif selon la revendication 2 ou 5, **caractérisé en ce que** ladite tige (51) est munie d'un conduit axial traversant (510), adapté pour relier des moyens de soufflage à ladite chambre cylindrique creuse (2), lesdits moyens de soufflage étant prévus à l'extérieur dudit dispositif (1) et adaptés pour pressuriser ou dépressuriser ce dernier.

7. Dispositif selon la revendication 3 ou 4, **caractérisé en ce que** lesdits premier et second trous traversants axiaux (50, 55) et lesdites première et seconde tiges (51, 56) sont filetés et mutuellement engagés, par le fait que lesdits moyens d'actionnement (53, 58) consistent en un premier et un second cliquet rotatif prévus dans les éléments de tête rigides relatifs (4A, 4B) et portant lesdits premier et second trous traversants axiaux filetés (50, 55), lesdits premier et second cliquets (53, 58) étant adaptés pour être tournés dans un sens ou dans l'autre, de manière indépendante, pour déterminer les décalages axiaux susmentionnés de l'élément de tête rigide correspondant (4A, 4B) les uns par rapport aux autres le long des tiges (51, 56), et **en ce que** lesdits accouplements filetés entre lesdits premier et second trous traversants axiaux (50, 55) et les tiges relatives (51, 56) sont adaptés pour stabiliser la position qui est atteinte à chaque fois par chaque élément de tête rigide (4A, 4B).

8. Dispositif selon la revendication 3, **caractérisé en ce que** au moins une desdites tiges (51, 56) est munie d'un conduit axial traversant (510, 560), adapté pour relier des moyens de soufflage à ladite chambre cylindrique creuse (2), lesdits moyens de soufflage étant prévus à l'extérieur du dispositif mentionné (1) et adaptés pour pressuriser ou dépressuriser ce dernier.

9. Dispositif selon la revendication 4, **caractérisé en ce que** au moins une desdites tiges (51, 56) est munie d'un conduit axial traversant (510, 560), adapté pour relier des moyens de soufflage, prévus à l'extérieur dudit dispositif (1) pour envoyer un fluide sous pression, à l'intérieur dudit soufflet (6), qui à son tour est affecté par des ouvertures pour le passage d'air sous pression vers l'intérieur de ladite chambre cylindrique creuse (2) destinée à être pressurisée.

10. Dispositif selon la revendication 4, **caractérisé en ce que** ladite chambre cylindrique creuse (2) est intégrée dans ledit manchon tressé (3), le premier étant élastique et le second étant défini par des filets sensiblement inextensibles (31, 32).
